(19) Europäisches Patentamt
European Patent Office
Office européen des brevets

(11) Veröffentlichungsnummer: **0 175 293**
**A1**

# (12) EUROPÄISCHE PATENTANMELDUNG

(21) Anmeldenummer: **85111561.8**

(22) Anmeldetag: **12.09.85**

(51) Int. Cl.⁴: **C 07 D 215/22**, C 07 D 223/16, C 07 D 401/12, C 07 D 405/12, C 07 D 413/12, A 61 K 31/47, A 61 K 31/55

(30) Priorität: **19.09.84 DE 3434271**

(43) Veröffentlichungstag der Anmeldung: **26.03.86 Patentblatt 86/13**

(84) Benannte Vertragsstaaten: **AT BE CH DE FR GB IT LI NL SE**

(71) Anmelder: **Beiersdorf Aktiengesellschaft, Unnastrasse 48, D-2000 Hamburg 20 (DE)**

(72) Erfinder: **Cohnen, Erich, Dr., Moorende 74, D-2155 Jork (DE)**
Erfinder: **Jacobitz, Petra, Dr., Isestrasse 54, D-2000 Hamburg 13 (DE)**

(54) **Bicyclische Lactame, Verfahren zu ihrer Herstellung und ihre Verwendung sowie diese Verbindungen enthaltende Zubereitungen.**

(57) Bicyclische Lactame der allgemeinen Formel I,

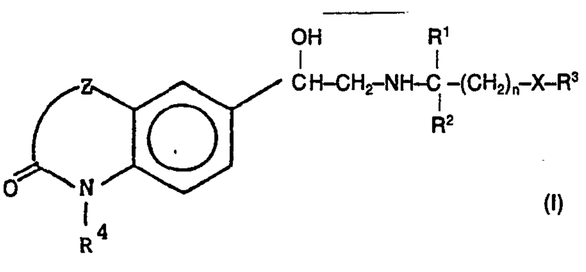

(I)

in der

$R^1$ und $R^2$, die gleich oder verschieden sein können, ein Wasserstoffatom oder eine Alkylgruppe mit 1 bis 3 Kohlenstoffatomen,

$R^3$ eine unsubstituierte Phenylgruppe oder eine, durch ein Halogenatom, eine oder zwei Alkoxygruppen, eine Methylendioxo-Gruppe, eine oder mehrere Hydroxo-Gruppen substituierte Phenylgruppe, eine Pyridylgruppe, eine Indolylgruppe, eine gegebenenfalls durch ein Halogenatom substituierte, 1,2-Benzisoxazolylgruppe, den Benzimidazol-2-on- oder 1,4-Benzodioxan-Rest,

$R^4$ ein Wasserstoffatom oder eine Alkylgruppe mit 1 bis 6 Kohlenstoffatomen, eine Carbalkoxymethylen-Gruppe,

Z eine unverzweigte, gesättigte oder ungesättigte Alkylengruppe mit 2 oder 3 Kohlenstoffatomen, die gegebenenfalls alkyliert ist,

X ein Sauerstoffatom oder eine Einfachbindung und

n die Zahlen 1, 2 oder 3 bedeuten sowie ihre tautomeren Formen und ihre Salze sowie Säureadditionssalze blokkieren sowohl die Alpha- als auch die Betarezeptoren des adrenergen Systems und sind daher zur Behandlung von Hypertonie, Durchblutungsstörungen, Angina pectoris und Coronarinsuffizienz geeignet.

EP 0 175 293 A1

BEIERSDORF AKTIENGESELLSCHAFT
HAMBURG

# Bicyclische Lactame
## Verfahren zu ihrer Herstellung und ihre Verwendung sowie diese Verbindungen enthaltende Zubereitungen

Gegenstand der Erfindung sind neue bicyclische Lactame der allgemeinen Formel I,

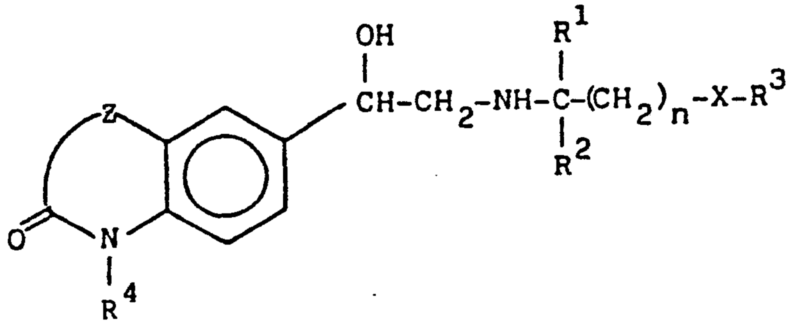

(I)

in der $R^1$ und $R^2$, die gleich oder verschieden sein können, ein Wasserstoffatom oder eine Alkylgruppe mit 1 bis 3 Kohlenstoffatomen,

$R^3$ eine unsubstituierte Phenylgruppe oder eine, durch ein Halogenatom, eine oder zwei Alkoxygruppen, eine Methylendioxo-Gruppe, eine oder mehrere Hydroxy-Gruppen substituierte Phenylgruppe, eine Pyridylgruppe, eine Indolylgruppe, eine gegebenenfalls

durch ein Halogenatom substituierte, 1,2-Benzisoxazolylgruppe, den Benzimidazol-2-on- oder 1,4-Benzodioxan-Rest,

$R^4$ ein Wasserstoffatom oder eine Alkylgruppe mit 1 bis 6 Kohlenstoffatomen, eine Carbalkoxymethylen-Gruppe,

Z eine unverzweigte, gesättigte oder ungesättigte Alkylengruppe mit 2 oder 3 Kohlenstoffatomen, die gegebenenfalls alkyliert ist,

X ein Sauerstoffatom oder eine Einfachbindung und

n die Zahlen 1, 2 oder 3 bedeuten

sowie ihre tautomeren Formen und ihre Salze sowie Säureadditionssalze, Verfahren zu ihrer Herstellung und ihre Verwendung sowie diese Verbindungen enthaltende Zubereitungen.

Eine Verbindung der Formel I ist in der Literatur beschrieben: Japan. Kokai 76,125,291 und Chem. Abstr. 87: 53 098r:

6-(2-Benzylamino-1-hydroxy)ethyl-3,4-dihydrocarbostyril

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Obgleich pharmazeutisch verträgliche Salze und Säureadditionssalze der neuen Verbindungen der Formel I und deren tautomere Formen bevorzugt sind, liegen alle Salze innerhalb des Bereichs der Erfindung. Alle Salze sind wertvoll zur Herstellung der Verbindungen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, beispielsweise durch Ionenaustauschverfahrensweisen, verwendet wird.

Die Verbindungen der allgemeinen Formel I und deren Salze enthalten asymmetrischen Kohlenstoffatome. Daher sind auch die verschiedenen optischen Isomeren sowie die Diastereoisomeren Gegenstand der Erfindung ebenso wie die Salze und Additionssalze dieser Verbindungen mit Säuren. Die Racemate können nach an sich bekannten Methoden in ihre optischen Antipoden aufgetrennt werden.

Die erfindungsgemäßen Alkylgruppen sowie die Alkylteile der Alkoxygruppen können geradkettig oder verzweigt sein und sind vorzugsweise Methyl-, Ethyl- und Propylgruppen.

Halogen ist Fluor, Chlor, Brom oder Jod, vorzugsweise Fluor, Chlor oder Brom, insbesondere aber Chlor.

Besonders bevorzugt werden Verbindungen der Formel I, in der mindestens $R^1$, insbesondere aber $R^1$ und $R^2$ Methyl bedeuten.

Der Rest $R^1$ ist vorzugsweise Wasserstoff, Methyl oder Ethyl. Weiterhin sind die Reste $R^1$ und $R^2$ vorzugsweise gleich und bedeuten Wasserstoff oder geradkettige Alkylgruppen, insbesondere Methyl- und Ethylgruppen. Auch gemischte Alkylsubstituenten $R^1$, $R^2$, insbesondere Methyl, Ethyl werden bevorzugt.

Bevorzugte Substituenten $R^3$ sind unsubstituierte Phenylgruppen oder wie angegeben substituierte Phenylgruppen, wobei Phenyl bevorzugt ist. Hydroxy-Phenylgruppen tragen vorzugsweise eine oder zwei Hydroxygruppen.

$R^4$ ist vorzugsweise Wasserstoff oder eine Alkylgruppe mit bis zu drei Kohlenstoffatomen, insbesondere Methyl oder Ethyl. Die Carbalkoxymethylengruppen besitzen 1 bis 6 Kohlenstoffatome, vorzugsweise 1 bis 3 Kohlenstoffatome im Alkoxyrest; besonders bevorzugt ist der Methoxy- oder Ethoxy-Rest.

Die ungesättigte Alkylengruppe Z enthält vorzugsweise eine olefinische Doppelbindung. Alkylierte Alkylengruppen Z besitzen eine bis drei, vorzugsweise eine, geradkettige oder verzweigte Alkylgruppe mit 1 bis 4 Kohlenstoffatomen, insbesondere Methyl- und/oder Ethylgruppen.

Bevorzugte Alkylengruppen Z sind:

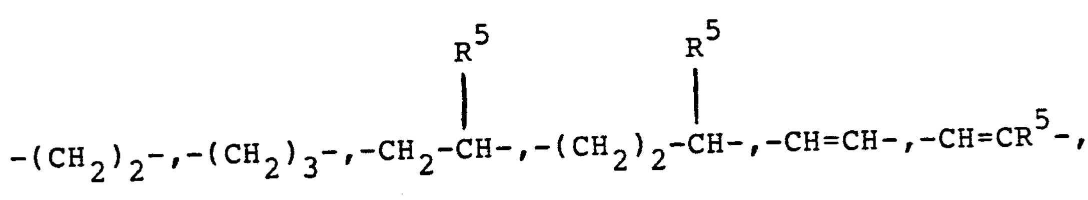

worin $R^5$ eine Alkylgruppe mit 1 bis 4 Kohlenstoffatomen, insbesondere Methyl und Ethyl bedeutet. Vorzugsweise befindet sich $R^5$ in der 4- oder 5-Stellung des Ringsystems. Bevorzugt werden gesättigte Alkylengruppen Z.

Besonders bevorzugt sind die 3,4-Dihydro-chinolin-2-on Verbindungen mit der Bedeutung Z $-(CH_2)_2-$ oder $-CH_2-CR^5H-$.

X ist vorzugsweise eine Einfachbindung und n bedeutet vorzugsweise die Zahl 2.

Bevorzugt werden weiterhin Verbindungen, die in der mit $-CR^1R^2-(CH_2)_n-$ gebildeten Alkylgruppe in der Formel I vorzugsweise mindestens 2 oder 4, insbesondere mindestens 5 und besonders bevorzugt genau 5 Kohlenstoffatome besitzen. Vorzugsweise ist in diesen Fällen n = 2. Weiterhin sind diese Verbindungen dann besonders bevorzugt, wenn $R^3$ Phenyl oder substituiertes Phenyl wie angegeben bedeutet, wobei Phenyl bevorzugt ist.

Besonders bevorzugte erfindungsgemäße Verbindungen der Formel I sind solche 3,4-Dihydro-chinolinone ($Z = -(CH_2)_2-$), bei denen $R^1$ und $R^2$ jeweils Methyl sind und n = 2 oder 3, insbesondere 2 ist und X eine Einfachbindung ist und $R^3$ Phenyl oder wie angegeben substituiertes Phenyl ist, wobei Phenyl bevorzugt wird.

Bevorzugte Reste $R^3$ sind 1,2-Benzisoxazolylgruppen. Sie können ein Halogenatom tragen oder unsubstituiert sein.

Benzazepin-Verbindungen mit $Z = -(CH_2)_3-$ und $-(CH_2)_2-CHR^5-$ werden bevorzugt.

Bedeutet X eine Einfachbindung, ist $R^3$ an die $-(CH_2)_n-$Gruppe gebunden.

Die folgenden erfindungsgemäßen Verbindungen und deren Salze und Säureadditionssalze mit hohem therapeutischen Effekt werden besonders bevorzugt, und zwar in der Form der Racemate sowie in der Form optisch aktiver Isomeren:

3,4-Dihydro-6-[1-hydroxy-2-[(1-methyl-3-phenyl-propyl)-amino]-ethyl]-chinolin-2(1H)-on

3,4-Dihydro-6-[1-hydroxy-2-[[1-methyl-2-(2-methoxy-phenoxy)-ethyl]-amino]-ethyl]-chinolin-2(1H)-on

3,4-Dihydro-6-[1-hydroxy-2-[[1-methyl-3-(6-chlor-1,2-benzisoxazol-3-yl)-propyl]-amino]-ethyl]-chinolin-2(1H)-on

3,4-Dihydro-6-[1-hydroxy-2-[[1,1-dimethyl-3-(2-methoxy-phenyl)-propyl]-amino]-ethyl]-chinolin-2(1H)-on

3,4-Dihydro-6-[1-hydroxy-2-[(1,1-dimethyl-3-phenyl-propyl)-amino]-ethyl]-chinolin-2(1H)-on

6-[1-Hydroxy-2-[[1,1-dimethyl-3-(2-methoxy-phenyl)-propyl]-amino]-ethyl]-chinolin-2(1H)-on

7-[1-Hydroxy-2-[(1,1-dimethyl-3-phenyl-propyl)-amino]-ethyl]-1,3,4,5,-tetrahydro-2H-1-benzazepin-2-on

Von diesen Verbindungen wird 3,4-Dihydro-6-[1-hydroxy-2-[(1,1-dimethyl-3-phenyl-propyl)-amino]-ethyl]-chinolin-2(1H)-on insbesondere bevorzugt. Es zeichnet sich durch eine besonders hohe Wirkungsstärke aus.

Die erfindungsgemäßen Verbindungen der Formel I sowie deren Isomere, ihre physiologisch verträglichen Salze und Säureadditionssalze sind therapeutische Wirkstoffe, besitzen hohe pharmakologische Wirkung und sind wertvolle Arzneimittel. Sie blockieren sowohl die Alpharezeptoren als auch die Betarezeptoren des adrenergen Systems und sind daher vorzugsweise zur Behandlung von Hypertonie, Durchblutungsstörungen, insbesondere peripheren Durchblutungsstörungen, Angina pectoris und Coronarinsuffizienz geeignet.

Die Kombination einer alpharezeptorenblockierenden Eigenschaft mit einer betarezptorenblockierenden ist aus zwei Gründen sinnvoll:

1. Im Gegensatz zu einer Betablockade allein wird der erhöhte Blutdruck vorwiegend durch die über Alpharezeptoren vermittelte Reduktion des peripheren Gefäßwiderstandes gesenkt; ein reflektorischer Anstieg des Herzzeitvolumens wird durch die betarezptorenblockierende Eigenschaft der Substanzen verhindert.

2. Die Aktivierung des Renin-Angiotensinsystems, die nach Vasodilatation durch Alphablockade beobachtet wird und einen blutdrucksteigernden Einfluß ausübt, wird durch Betablockade unterdrückt.

Die in vorliegender Erfindung genannten Verbindungen senken bei Kaninchen in einer Dosis von 10 bis 100 mg/kg p.o. die Isoprenalin-bedingte Tachycardie

Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 10 bis 50 mg.

um 50 % ($beta_1$-Blockade) und den Phenylephrin-bedingten Blutdruckanstieg um 50 % ($alpha_1$-Blockade).

Die Verbindungen der vorliegenden Erfindung können oral oder parenteral angewendet werden. Die Einzeldosis beträgt beim Menschen 1 mg bis 500 mg, vorzugsweise 10 mg bis 100 mg, insbesondere 30 mg bis 50 mg. Diese Dosierungen sind vorteilhaft zur Behandlung der vorstehend genannten Krankheiten, insbesondere zur Behandlung von Hypertonie.

Die Tagesdosis ist, wie für Alpha- und Betarezeptoren üblich, individuell abzustimmen, weil sie von der Rezeptorenempfindlichkeit und dem Sympathikotonus des Patienten abhängt. Zweckmäßigerweise wird die Behandlung mit niederen Dosen begonnen und dann gesteigert.

Gemäß der Erfindung werden pharmazeutische Zusammensetzungen geschaffen, die eine Verbindung der Formel I oder deren pharmazeutisch verträglichen Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten oder parenteral in Form von Lösungen oder Suspensionen verabreicht werden. Oral zu verabreichende Präparate können ein oder mehrere Zusätze, wie Süßungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, zum

Ein erstes Verfahren zur Herstellung der Verbindungen der Formel I ist dadurch gekennzeichnet, daß man Halogenketone der allgemeinen Formel II

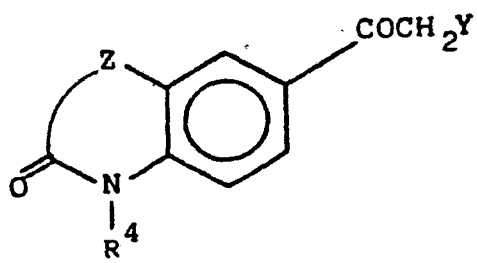

(II)

in der $R^4$ und Z die angegebene Bedeutung haben und Y ein Chlor oder Bromatom bedeutet, mit einem Amin $R^6R^7$-NH, bei dem $R^6$ ein Wasserstoffatom oder eine Benzylgruppe und $R^7$ die Gruppe

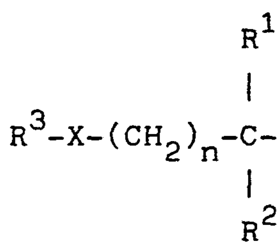

bedeutet, wobei $R^1$, $R^2$, $R^3$, X und n wie oben definiert sind, zu den Aminoketonen der allgemeinen Formel III umsetzt:

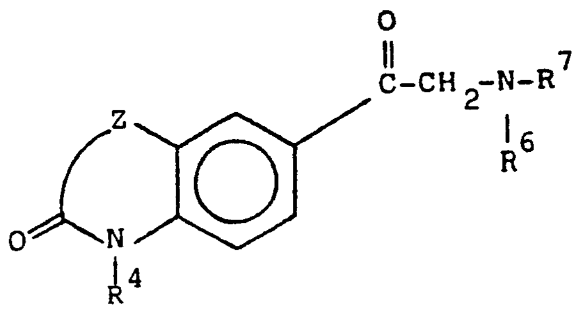

(III)

Verbindungen der Formel III, in der $R^6$ eine Benzylgruppe darstellt, werden durch katalytische Hydrierung in die erfindungsgemäßen Verbindungen überführt und Verbindungen der Formel III, in der $R^6$ ein Wasserstoffatom bedeutet, werden durch Reduktion der Ketogruppe mit komplexen Metallhydriden oder katalytische Hydrierung in Verbindungen der Formel I umgewandelt.

Die Umsetzung der Halogenketone mit Aminen erfolgt in einem geeigneten Lösungsmittel wie Dimethylformamid, Acetonitril, tert.-Butanol in Gegenwart eines tert.-Amins wie Triäthylamin, Pyridin oder eines säurebindenden Mittels wie z.B. Natriumcarbonat.

Die Reduktion des Ketons der Formel (III) wird in einem Alkohol wie Methanol oder Ethanol mit komplexen Metallhydriden wie Natriumborhydrid oder durch katalytische Reduktion mit Wasserstoff in Gegenwart von Edelmetallkatalysatoren wie Platin, Palladium oder Raney-Nickel durchgeführt. Eine gegebenenfalls vorhandene Benzyl-Schutzgruppe ($R^6$ = $CH_2C_6H_5$) wird bei katalytischer Reduktion hydrogenolytisch abgespalten.

Ein weiteres Verfahren zur Herstellung der Verbindungen der Formel I ist dadurch gekennzeichnet, daß man einen Aminoalkohol der Formel IV

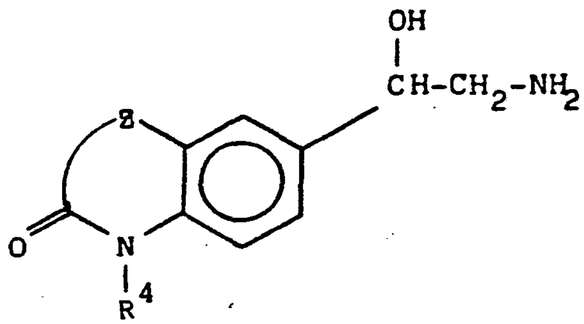

(IV)

mit der angegebenen Bedeutung von $R^4$ und Z, der durch Reaktion eines Halogenketons der Formel II mit Dibenzylamin und anschließende katalytische Hydrierung gewonnen werden kann, mit einem geeigneten Aldehyd oder Keton der allgemeinen Formel V

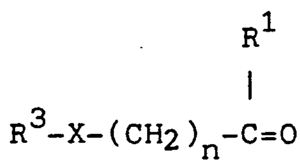

(V)

in der $R^1$, $R^3$, X und n die angegebene Bedeutung haben, kondensiert und das intermediär gebildete Azomethin mit einem komplexen Metallhydrid oder durch katalytische Reduktion mit Wasserstoff in die erfindungsgemäßen Verbindungen der Formel I überführt.

Die Kondensation des Amins der Formel (IV) mit einem Keton oder Aldehyd (V) erfolgt in geeigneten Lösungsmitteln wie Alkoholen bei Temperaturen von 20 - 100°C. Die Reduktion des Azomethins erfolgt im gleichen Lösungsmittel durch komplexe Metallhydride, vorzugsweise Natriumborhydrid, oder katalytische Reduktion mit Wasserstoff und Katalysatoren wie Raney-Nickel, Palladium oder Platin bei Raumtemperatur.

Ein weiteres Verfahren zur Herstellung der Verbindungen der Formel I ist dadurch gekennzeichnet, daß man einen alpha-Ketoaldehyd der Formel VI

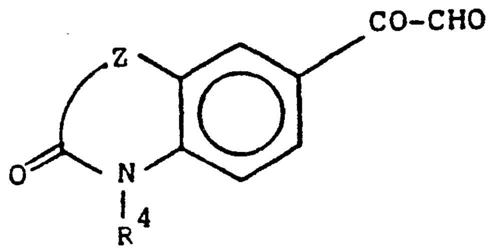

(VI)

in der $R^4$ und Z die angegebene Bedeutung haben, mit einem Amin der Formel VII

$$R^3\text{-X-}(CH_2)_n\text{-}\overset{\displaystyle R^1}{\underset{\displaystyle R^2}{\overset{|}{\underset{|}{C}}}}\text{-}NH_2 \qquad \text{(VII)}$$

in der $R^1$, $R^2$, $R^3$, X und n die angegebene Bedeutung haben, umsetzt und die erhaltene Azomethin-Zwischenstufe mit komplexen Metallhydriden oder auf katalytischem Wege mit Wasserstoff reduziert und auf diese Weise in die erfindungsgemäßen Verbindungen der Formel I überführt.

Die Kondensation des Amins der Formel VII mit einem Ketoaldehyd (VI) erfolgt in geeigneten Lösungsmitteln wie Alkoholen bei Temperaturen von 20 - 100°C. Die Reduktion des Azomethins erfolgt im gleichen Lösungsmittel durch komplexe Metallhydride, vorzugsweise Natriumborhydrid, oder katalytische Reduktion mit Wasserstoff und Katalysatoren wie Raney-Nickel, Palladium oder Platin bei Raumtemperatur.

Die Ausgangsverbindungen sind bekannt oder können nach bekannten Verfahren erhalten werden.

Die Verbindungen der allgemeinen Formel I können sowohl Basen als auch Säuren, beziehungsweise amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Halogenwasserstoffsäuren, zum Beispiel Salzsäure oder Schwefelsäure, und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt und man erhält nach Etherzusatz das Salz. Bevorzugte Salze sind die Alkali-, Erdalkali- und Ammoniumsalze der Verbindungen der Formel I, die mit den entsprechenden Basen, insbesondere Natrium-, Kalium- oder Ammoniumhydroxid erhalten werden.

Diastereoisomere können in bekannter Weise aufgrund der physikalisch-chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit optisch aktiven Säuren oder Basen, die mit der racemischen Verbindung ein Salz bilden, Trennung der Diastereoisomeren durch fraktionierte Kristallisation und Freisetzung der Enantiomeren durch geeignete Mittel. Besonders geeignete optisch aktive Säuren sind beispielsweise die d- und l-Formen der Weinsäure, Ditoluylweinsäure, Äpfelsäure, Mandelsäure, Kamphersulfonsäure oder Pyrrolidon-carbonsäure. Geeignete optisch aktive Basen

sind alpha-Phenyläthylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele dienen zur Erläuterung der Erfindung:

## Beispiel 1

3,4-Dihydro-6-/1-hydroxy-2-/(1,1-dimethyl-3-phenyl-propyl)-amino/-ethyl/-chinolin-2(1H)-on

Eine Lösung von 1,4 g (0.006 mol) 3,4-Dihydro-6-(alpha,alpha-dihydroxy-acetyl)-chinolin-2(1H)-on und 1,37 g (0,008 mol) 1,1-Dimethyl-3-phenyl-propanamin in 60 ml Ethanol und 30 ml Dimethylformamid wird 2 Stunden auf 80°C erhitzt und nach Abkühlen auf Raumtemperatur mit ca. 1,5 g Natriumborhydrid nach und nach versetzt. Nach 2 Stunden wird mit Essigsäure angesäuert und das Lösungsmittel im Vakuum abgedampft. Der Rückstand wird nach Alkalisieren mit 2N Natronlauge mit Methylenchlorid extrahiert. Der Rückstand der organischen Phase wird durch Säulenchromatografie an Kieselgel gereinigt ($CHCl_3$/MeOH 93/7).

Mit äthanolischer Salzsäure erhält man nach Umkristallisieren aus Isopropanol 1,25 g 3,4-Dihydro-6-/1-hydroxy-2-/(1,1-dimethyl-3-phenyl-propyl)-amino/-ethyl/-chinolin-2(1H)-on-Hydrochlorid. Fp. 231-232°C (Z.)

Analog Beispiel 1 wurden die folgenden Verbindungen der Formel I hergestellt (X = - bedeutet eine Einfachbindung), worin $R^4$ und $R^5$ Wasserstoff sind und Z $-(CH_2)_2-$ bedeutet (3,4-Dihydro-chinolin-2-on-Verbindungen):

| Beispiel | $R^1$ | $R^2$ | $R^3$ | X | n | Salz | Fp. °C |
|---|---|---|---|---|---|---|---|
| 2 | $CH_3$ | $CH_3$ | 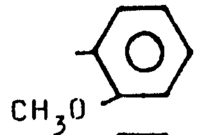 | - | 2 | Fumarat<br>Hydrochlorid | 207-209<br>182 (Z) |
| 3 | $CH_3$ | $CH_3$ | 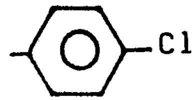 | - | 2 | Hydrochlorid | 180 (Z) |
| 4. | $CH_3$ | $CH_3$ | 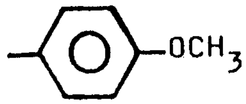 | - | 2 | Hydrochlorid | 238 (Z) |
| 5 | $CH_3$ | $CH_3$ | 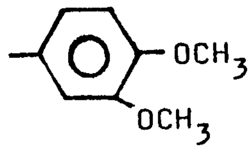 | - | 2 | | |
| 6 | $CH_3$ | $CH_3$ | 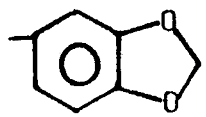 | - | 2 | | |
| 7 | $CH_3$ | $CH_3$ | 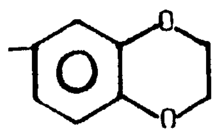 | - | 2 | Hydrochlorid | 195 |
| 8 | $CH_3$ | $CH_3$ | 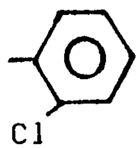 | - | 2 | | |

| Beispiel | $R^1$ | $R^2$ | $R^3$ | X | n | Salz | Fp. °C |
|---|---|---|---|---|---|---|---|
| 9 | $CH_3$ | $CH_3$ | 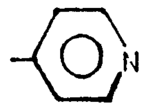 | - | 2 | Dihydro-chlorid | 204 (Z.) |
| 10 | $CH_3$ | $CH_3$ | 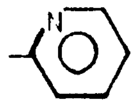 | - | 2 | Dihydro-chlorid | 195-196 (Z.) |
| 11 | $CH_3$ | $CH_3$ | 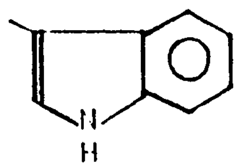 | - | 1 | Hydro-chlorid | 254-256 |
| 12 | $CH_3$ | $CH_3$ | 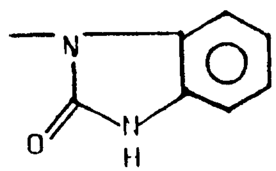 | - | 3 | Hydro-chlorid | amorph |
| 13 | $CH_3$ | $CH_3$ | 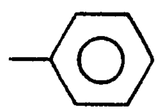 | - | 3 | Base | 170° |

In den folgenden Beispielen ist mit $R^5$ auch die Stellung dieses Substituenten am Ringsystem bei jeweils gegebenem Z mit diesem Substituenten $R^5$ sowie $R^4$ angegeben.

| Beispiel | $R^1$ | $R^2$ | $R^3$ | $R^4$ | $R^5$ | X | Z | n | Salz | Fp. °C |
|---|---|---|---|---|---|---|---|---|---|---|
| 14 | $CH_3$ | $CH_3$ | 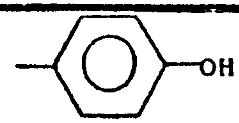 | H | H | - | $(CH_2)_2$ | 2 | HCl | 219 |
| 15 | $CH_3$ | $CH_3$ | 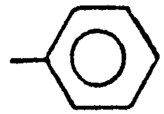 | H | 4-$CH_3$ | - | $-CH_2-CH(CH_3)-$ | 2 | HCl | 212 |
| 16 | $CH_3$ | $CH_3$ | 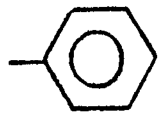 | $CH_3$ | H | - | $(CH_2)_2$ | 2 | HCl | 227 |
| 17 | $CH_3$ | $CH_3$ | 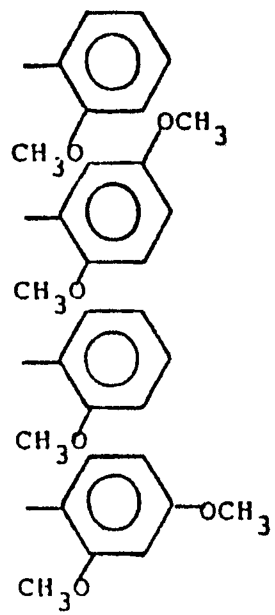 | H | H | - | $(CH_2)_3$ | 2 | HCl | 210 |
| 18 | $CH_3$ | $CH_3$ | | H | H | - | $(CH_2)_2$ | 2 | HCl | amorph |
| 19 | $CH_3$ | $CH_3$ | | H | H | - | $(CH_2)_2$ | 3 | HCl | 156 |
| 20 | $CH_3$ | $CH_3$ | | H | H | - | $(CH_2)_2$ | 2 | HCl | amorph |

| Beispiel | $R^1$ | $R^2$ | $R^3$ | $R^4$ | $R^5$ | X | Z | n | Salz | Fp. °C |
|---|---|---|---|---|---|---|---|---|---|---|
| 21 | $CH_3$ | $CH_3$ | 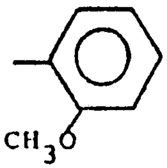 | H | H | - | -CH=CH- | 2 | | |
| 22 | $CH_3$ | $CH_3$ | 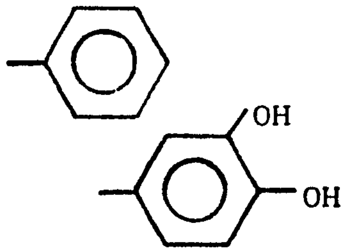 | $CH_2COOCH_3$ | H | - | $(CH_2)_2$ | 2 | HCl | 230 |
| 23 | $CH_3$ | $CH_3$ | | H | H | - | $(CH_2)_2$ | 2 | | |

Beispiel 24

3,4-Dihydro-6-[1-hydroxy-2-[[1-methyl-3-(1,3-benzodioxol-5-yl)-propyl]-amino]-ethyl]-chinolin-2(1H)-on

Eine Suspension von 3,0 g (0,012 mol) 3,4-Dihydro-6-(2-amino-1-hydroxyethyl)-chinolin-2(1H)-on-Hydrochlorid in Ethanol wird mit Natriumethylatlösung (0.013 mol) neutralisiert und nach Zusatz von 4,60 g (0.024 mol) 4-(1,3-Benzodioxol-5-yl)-butan-2-on 2 Stunden zum Sieden erhitzt. Nach Abkühlung werden portionsweise 2,3 g Natriumborhydrid zugesetzt, dann wird eine Stunde bei Raumtemperatur gerührt und mit Essigsäure angesäuert. Das Lösungsmittel wird im Vakuum entfernt und der Rückstand wird mit 2N Natronlauge versetzt. Man extrahiert mit Methylenchlorid, dampft die organische Phase ein und überführt den Rückstand mit äthanolischer Salzsäure in das Hydrochlorid des 3,4-Diyhdro-6-[1-hydroxy-2-[[1-methyl-3-(1,3-benzodioxol-5-yl)-propyl]-amino]-ethyl]-chinolin-2(1H)-ons.

Nach Umkristallisieren aus Isopropanol erhält man 1,2 g vom Fp. 187-190°C (Z.)

Zur gleichen Verbindung gelangt man auch durch katalytische Hydrierung des aus dem Amin und Keton intermediär gebildeten Azomethins mittels Wasserstoff bei Raumtemperatur und 10 bar in Gegenwart von Palladium-Kohle (10 %).

Analog Beispiel 24 werden die folgenden Verbindungen der Formel I synthetisiert (X = - bedeutet eine Einfachbindung), worin $R^4$ und $R^5$ Wasserstoff sind und Z $-(CH_2)_2-$ bedeutet (3,4-Dihydro-chinolin-2-on-Verbindungen):

| Beispiel | $R^1$ | $R^2$ | $R^3$ | X | n | Salz | Fp. °C |
|---|---|---|---|---|---|---|---|
| 25 | H | $CH_3$ | 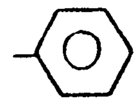 | - | 2 | Hydrochlorid | 169-171 (Z.) |
| 26 | H | $CH_3$ | 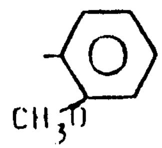 | O | 1 | Hydrochlorid | 158-159 (Z.) |
| 27 | H | $CH_3$ | 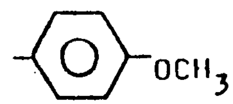 | - | 1 | Hydrochlorid | 174 |
| 28 | H | H | 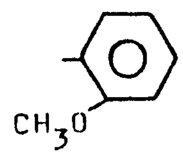 | O | 1 | Hydrochlorid | 156-158 (Z.) |
| 29 | H | $CH_3$ | 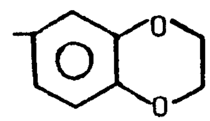 | - | 2 | | |
| 30 | H | $CH_3$ | 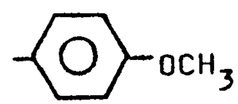 | - | 2 | Hydrochlorid | 209 (Z.) |
| 31 | H | $CH_3$ | 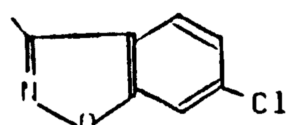 | - | 2 | Hydrochlorid | 197-198 (Z.) |
| 32 | H | $CH_3$ | 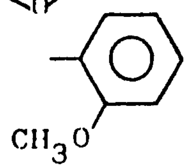 | - | 2 | Hydrochlorid | 178-180 (Z.) |

Beispiel 33

3,4-Dihydro-6-[1-hydroxy-2-[[1,1-dimethyl-3-(2-methoxy-phenyl)-propyl]-amino]-ethyl]-chinolin-2(1H)-on

6,0 g (0,023 mol) 3,4-Dihydro-6-bromacetyl-chinolin-2(1H)-on, 4,8 g (0,025 mol) 1,1-Dimethyl-3-(2-methoxy-phenyl)-propanamin und 3,2 g (0,03 mol) Kaliumcarbonat werden in 100 ml Dimethylformamid 24 Stunden bei Raumtemperatur gerührt. Nach Verdünnen mit Methanol wird mit einem Überschuß Natriumborhydrid reduziert. Die Lösungsmittel werden im Vakuum entfernt, der Rückstand wird in Methylenchlorid aufgenommen und mit Wasser extrahiert. Nach Säulenchromatografie an Kieselgel ($CHCl_3$/MeOH 90/10) erhält man 3,4-Dihydro-6-[1-hydroxy-2-[[1,1-dimethyl-3-(2-methoxy-phenyl)-propyl]-amino]-ethyl]-chinolin-2(1H)-on als Base, die in üblicher Weise in das Hydrochlorid überführt wird. Fp. 180°C (Z.)

Beispiel 34

Herstellung von Tabletten

Tabletten, welche die nachstehend angegebenen Bestandteile enthalten, werden nach bekannten Arbeitsweisen hergestellt. Diese sind für die Behandlung von den vorstehend genannten Krankheiten, insbesondere Hypertonie, in einer Dosierungsmenge von 40 mg einmal bis zweimal täglich geeignet.

| | Tablette A | Tablette B |
|---|---|---|
| 3,4-Dihydro-6-/1-hydroxy-2-/ /1-methyl-2-(2-methoxy-phenoxy)-ethyl7-amino7-ethyl7-chinolin-2(1H)-on-Hydrochlorid | 40 mg | 20 mg |
| Lactose | 90 mg | 90 mg |
| Maisstärke | 5 mg | 5 mg |
| Magnesiumstearat | 1 mg | 1 mg |

Beispiel 35

Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt.

| | |
|---|---|
| 3,4-Dihydro-6-/1-hydroxy-2-/(1-methyl-3-phenyl-propyl)-amino7-ethyl7-chinolin-2(1H)-on-Hydrochlorid | 10 mg |
| Natriumchlorid | 18 mg |
| dest. Wasser ad | 2,0 ml |

Beispiel 36

Herstellung von Tabletten

Tabletten, welche die nachstehend angegebenen Bestandteile enthalten, werden nach bekannten Arbeitsweisen hergestellt. Diese sind für die Behandlung von den vorstehend genannten Krankheiten, insbesondere Hypertonie, in einer Dosierungsmenge von 30 mg einmal bis zweimal täglich geeignet.

| | Tablette A | Tablette B |
|---|---|---|
| 3,4-Dihydro-6-/1-hydroxy-2-/(1,1,-dimethyl-3-phenyl-propyl)-amino/-ethyl/-chinolin-2(1H)-on | 30 mg | 10 mg |
| Lactose | 90 mg | 90 mg |
| Maisstärke | 5 mg | 5 mg |
| Magnesiumstearat | 1 mg | 1 mg |

Patentansprüche

1. Bicyclische Lactame der allgemeinen Formel I,

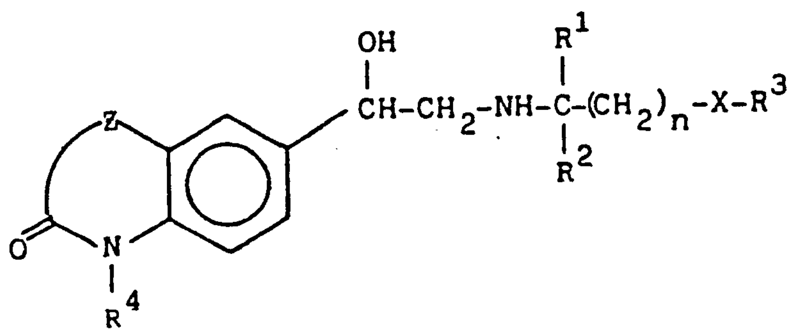

(I)

in der $R^1$ und $R^2$, die gleich oder verschieden sein können, ein Wasserstoffatom oder eine Alkylgruppe mit 1 bis 3 Kohlenstoffatomen,

$R^3$ eine unsubstituierte Phenylgruppe oder eine, durch ein Halogenatom, eine oder zwei Alkoxygruppen, eine Methylendioxo-Gruppe, eine oder mehrere Hydroxy-Gruppen substituierte Phenylgruppe, eine Pyridylgruppe, eine Indolylgruppe, eine gegebenenfalls durch ein Halogenatom substituierte, 1,2-Benzisoxazolylgruppe, den Benzimidazol-2-on- oder 1,4-Benzodioxan-Rest,

$R^4$ ein Wasserstoffatom oder eine Alkylgruppe mit 1 bis 6 Kohlenstoffatomen, eine Carbalkoxymethylen-Gruppe,

Z eine unverzweigte, gesättigte oder ungesättigte Alkylengruppe mit 2 oder 3 Kohlenstoffatomen, die gegebenenfalls alkyliert ist,

X ein Sauerstoffatom oder eine Einfachbindung und

n die Zahlen 1, 2 oder 3 bedeuten,

sowie ihre tautomeren Formen und ihre Salze sowie Säureadditionssalze, ausgenommen

6-(2-Benzylamino-1-hydroxy)ethyl-3,4-dihydrocarbostyril.

2. Verbindungen gemäß Anspruch 1, dadurch gekennzeichnet, daß mindestens $R^1$, insbesondere aber $R^1$ und $R^2$ Methyl bedeuten.

3. Verbindungen gemäß Anspruch 1, dadurch gekennzeichnet, daß die Alkylengruppe Z

$$-(CH_2)_2-, -(CH_2)_3-, -CH_2-\overset{\displaystyle R^5}{\overset{|}{C}}H-, -(CH_2)_2-\overset{\displaystyle R^5}{\overset{|}{C}}H-, -CH=CH-, -CH=CR^5-$$

bedeutet,

worin $R^5$ eine Alkylgruppe mit 1 bis 4 Kohlenstoffatomen bedeutet.

4. 3,4-Dihydro-6-[1-hydroxy-2-[(1,1-dimethyl-3-phenylpropyl)-amino]-ethyl]-chinolin-2(1H)-on.

5. Verfahren zur Herstellung der Verbindungen der Formel I, dadurch gekennzeichnet, daß man

Halogenketone der allgemeinen Formel II

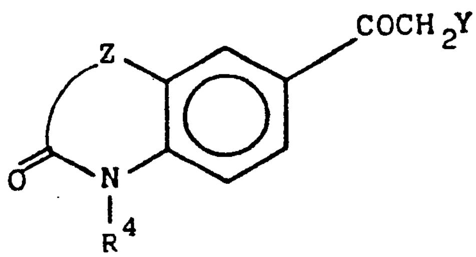

(II)

in der $R^4$ und Z die angegebene Bedeutung haben und in der Y ein Chlor- oder Bromatom bedeutet, mit einem Amin $R^6R^7$-NH, bei dem $R^6$ ein Wasserstoffatom oder eine Benzylgruppe und $R^7$ die Gruppe

$$R^3-X-(CH_2)_n-\overset{\displaystyle R^1}{\underset{\displaystyle R^2}{\overset{|}{\underset{|}{C}}}}-$$

bedeutet, wobei $R^1$, $R^2$, $R^3$, X und n wie oben definiert sind, zu den Aminoketonen der allgemeinen Formel III umsetzt,

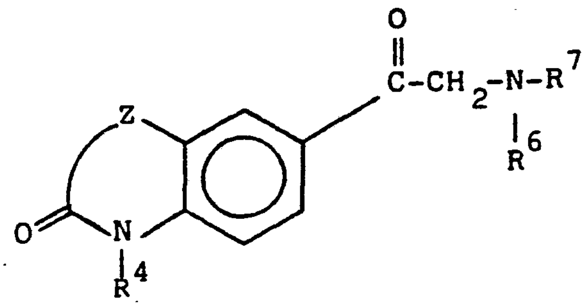

(III)

und Verbindungen der Formel III, in der $R^6$ eine Benzylgruppe darstellt, durch katalytische Hydrierung in die erfindungsgemäßen Verbindungen überführt und Verbindungen der Formel III in der $R^6$ ein Wasserstoffatom bedeutet,durch Reduktion der Ketogruppe mit komplexen Metallhydriden oder katalytische Hydrierung in Verbindungen der Formel (I) überführt.

6. Verfahren zur Herstellung der Verbindungen der Formel I,in der $R^2$ Wasserstoff bedeutet, dadurch gekennzeichnet, daß maneinen Aminoalkohol der Formel IV

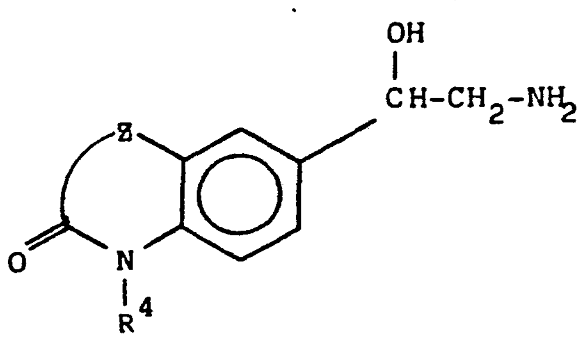

(IV)

mit der angegebenen Bedeutung von $R^4$ und Z

mit einem Aldehyd oder Keton der allgemeinen Formel V

$$\begin{array}{c} \qquad\qquad\qquad R^1 \\ \qquad\qquad\qquad | \\ R^3-X-(CH_2)_n-C=O \end{array} \qquad (V)$$

in der $R^1$, $R^3$, X und n die angegebene Bedeutung haben, kondensiert und das intermediär gebildete Azomethin mit einem komplexen Metallhydrid oder durch katalytische Reduktion mit Wasserstoff in die erfindungsgemäßen Verbindungen der Formel I überführt.

7. Verfahren zur Herstellung der Verbindungen der Formel I, dadurch gekennzeichnet, daß man einen alpha-Ketoaldehyd der Formel VI

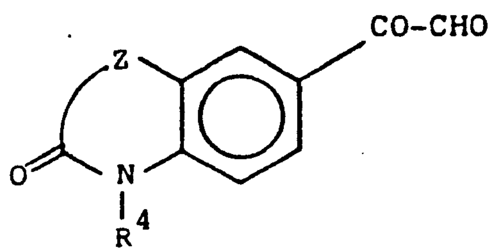

(VI)

in der $R^4$ und Z die angegebene Bedeutung haben, mit einem Amin der Formel VII

$$\begin{array}{c} \qquad\qquad\qquad R^1 \\ \qquad\qquad\qquad | \\ R^3-X-(CH_2)_n-C-NH_2 \\ \qquad\qquad\qquad | \\ \qquad\qquad\qquad R^2 \end{array} \qquad (VII)$$

in der $R^1$, $R^2$, $R^3$, X und n die angegebene Bedeutung haben, umsetzt und die erhaltene Azomethin-Zwischenstufe mit komplexen Metallhydriden oder auf katalytischem Wege mit Wasserstoff reduziert und auf diese Weise in die erfindungsgemäßen Verbindungen der Formel I überführt.

8. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

9. Verwendung von Verbindungen der Formel I gemäß Anspruch 1 als therapeutische Wirkstoffe, insbesondere zur Behandlung von Hypertonie, Durchblutungsstörungen, peripheren Durchblutungsstörungen, Angina pectoris und Coronarinsuffizienz.

0 175 293

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

EP 85111561.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | CHEMICAL ABSTRACTS, Band 87, Nr. 7, 15. August 1977, Columbus, Ohio, USA<br>K. NAKGAWA "Carbostyril derivatives" Seite 453, 1. Spalte, Referat 53 098r<br>& JP-Kokai-Nr. 125 291/76<br>-- | 1,3,5, 8 | C 07 D 215/22<br>C 07 D 223/16<br>C 07 D 401/12<br>C 07 D 405/12<br>C 07 D 413/12<br>A 61 K 31/47<br>A 61 K 31/55 |
| A | US - A - 4 348 398 (ATKINSON)<br>* Ansprüche 1,12 *<br>-- | 1,8 | |
| A | US - A - 3 975 391 (NA KAGAWA)<br>* Anspruch 1; Zusammenfassung *<br>-- | 1,8 | |
| A | EP - A1 - 0 011 747 (BASF)<br>* Zusammenfassung *<br>-- | 1,8 | |

**RECHERCHIERTE SACHGEBIETE (Int. Cl.4)**

C 07 D 215/00
C 07 D 223/00
C 07 D 401/00
C 07 D 405/00
C 07 D 413/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8
Unvollständig recherchierte Patentansprüche: –
Nicht recherchierte Patentansprüche: 9
Grund für die Beschränkung der Recherche:

Verfahren zur therapeutischen Behandlung des menschlichen oder tierischen Körpers (Artikel 52(4), EPÜ).

| Recherchenort | Abschlußdatum der Recherche | Prüfer |
|---|---|---|
| WIEN | 17-12-1985 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

0 175 293

Europäisches Patentamt

# EUROPÄISCHER TEILRECHERCHENBERICHT

EP 85111561.8

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | DE - A - 1 620 124 (PFIZER)<br>---- | | |
| | | | RECHERCHIERTE SACHGEBIETE (Int. Cl.4) |
| | | | |